# EUROPEAN PATENT APPLICATION

(11) **EP 4 216 166 A1**
(43) Date of publication of application: **26.07.2023**
(21) Application number: 22305064.2
(22) Date of filing: 21.01.2022
(51) Int. Cl.: G06T 11/00

(54) **METHOD AND SYSTEM FOR RECONSTRUCTING A 3D MEDICAL IMAGE**

(71) Applicant: Ecential Robotics, 38610 Gieres (FR)
(72) Inventor: LAVALLEE, Stéphane, 38610 GIERES (FR); ARMAND, David, 38610 GIERES (FR); PIERRE, Arnaud, 38610 GIERES (FR); VULLIET, Clémentine, 38610 GIERES (FR)
(74) Representative: Regimbeau

(57) **Abstract**

The invention relates to a method for reconstructing a 3D medical image of a region of interest from a set of 2D X-ray images acquired by an X-ray imaging system comprising an X-ray source (S) and an image detector (D), comprising the steps of:
- computing n paths of the X-ray imaging system, n being an integer greater than or equal to 2, said paths being adapted to acquire n respective subsets of 2D X-ray images together forming the set of 2D X-ray images, each image (I_{A}, I_{B}, I_{C}) of a subset defining a respective cone of projection (C_{A}, C_{B}, C_{C}) of the region of interest onto the image detector, such that, for a same position of the X-ray source (S) along the n paths, the cone of projection of an image of any subset is contiguous to the cone of projection of an image of at least one other subset;
- implementing the computed n paths to acquire the n subsets of images;
- reconstructing the 3D medical image by intersecting the cones of projection defined by each image of the n subsets.

## Description

### TECHNICAL FIELD

The present disclosure relates to a method and a system for reconstructing a 3D medical image.

### TECHNICAL BACKGROUND

X-Ray imaging systems are frequently used during medical surgical procedures to provide physicians with image-based information about a patient's anatomical situation and/or the position and orientation of surgical instruments.

Such X-ray imaging systems typically provide two-dimensional (2D) projection images with different anatomical structures superimposed along the path of the X-rays.

A typical example of such a system for use in an intra-operative setting is the so-called C-arm which comprises a base frame on which a C-shaped arm is attached with several intermediate joints allowing moving the C-shaped arm in space along several degrees of freedom. One end of the C-shaped arm carries an X-ray source and the other end carries an image detector.

Due to the limited information provided by these 2D images, three-dimensional (3D) imaging techniques have become necessary over the past decades.

While computer tomography is a well-established class of stationary X-ray imaging systems used for 3D reconstruction in a radiology department, these systems are in general not usable inside an operating room.

Recent years have seen an increasing interest in tomographic reconstruction techniques, also known as cone-beam reconstruction techniques (CBCT), using two-dimensional detectors.

Special efforts have been made to enable the aforementioned C-arms to provide three-dimensional information by automatically acquiring a set of 2D images and subsequently reconstructing a 3D image reconstruction based on said cone-beam reconstruction techniques.

An inherent problem with this imaging modality is the limitation in size of the reconstructed 3D image. The factors limiting this size of reconstruction are notably:
- the surface of the detection panel on the C-arm;
- the characteristic dimensions of the C-arm, which include in particular the distance between the X-ray source and the detection panel;
- the range of mobility of the C-arm along its degrees of freedom;
- the low number of degrees of freedom in the mobility of the C-arm and/or its components (detection panel notably), restraining the complexity of the path of the C-arm for 2D images acquisitions.

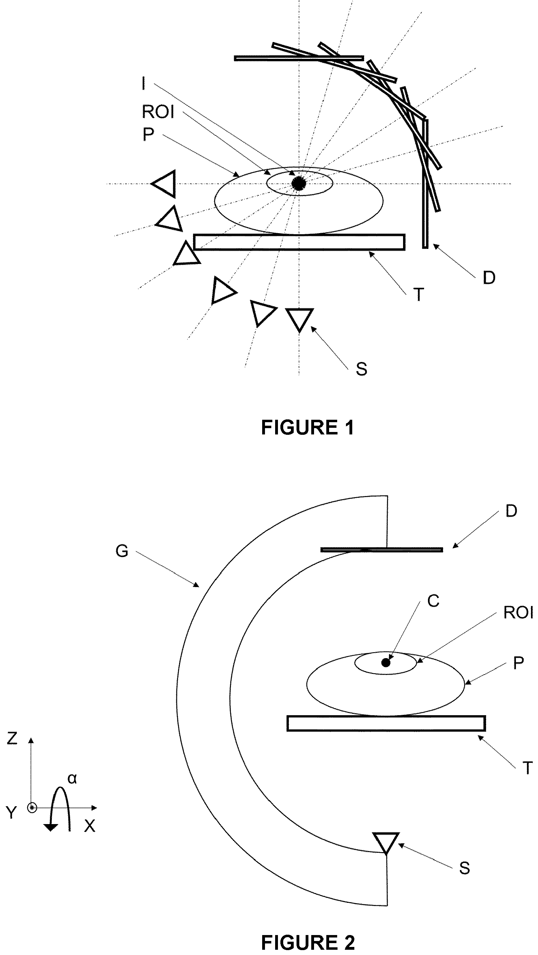

FIG. 1 is a schematic representation of an orbital path of a C-arm as commonly practiced in the prior art. Several positions of the X-ray source S and the detector D along half the orbital path (i.e. along a 90° angular sector) are represented, the dotted lines linking one position of the X-ray source S to the corresponding position of the detector D representing the axis of revolution of the X-ray emission cone of the X-ray source during the acquisition of a 2D X-ray image. The patient P is lying on a table T. The path of the C-arm is orbital, meaning that the C-arm rotates about an axis which is perpendicular to the plane of the C-arm (which corresponds to the plane of the sheet on which FIG. 1 is drawn), over a 180° angular sector. In said plane, the center of rotation of the C-arm is the isocenter I of the C-arm, which is the center of the segment which links the X-ray source S to the center of the detector panel D. To reconstruct a 3D image of a region of interest ROI of the patient, a plurality of 2D X-ray images of the region of interest are acquired during the orbital path of the C-arm.

However, it is often desirable, during minimally invasive surgery, to have a reconstructed 3D image as large as possible, not only for the surgeon's comfort but also for better outputs in certain surgeries: for example for interventions on several bones (several vertebrae for example), or for surgery on obese patients.

### SUMMARY OF THE DISCLOSURE

It is thus desirable to optimize the path of an X-ray imaging system during the acquisition of a set of 2D images in view of generating a reconstructed 3D image of a region of interest (ROI) of a patient as large as possible within safety parameters.

In some embodiments, the invention thus provides a method for reconstructing a 3D medical image of a region of interest from a set of 2D X-ray images acquired by an X-ray imaging system comprising an X-ray source and an image detector, comprising the steps of:
- computing n paths of the X-ray imaging system, n being an integer greater than or equal to 2, said paths being adapted to acquire n respective subsets of 2D X-ray images together forming the set of 2D X-ray images, each image of a subset defining a respective cone of projection of the region of interest onto the image detector, such that, for a same position of the X-ray source along the n paths, the cone of projection of an image of any subset is contiguous to the cone of projection of an image of at least one other subset;
- implementing the computed n paths to acquire the n subsets of images;
- reconstructing the 3D medical image by intersecting the cones of projection defined by each image of the n subsets.

By "contiguous" is meant in the present text that two adjacent cones of projection corresponding to a same position of the X-ray source along the respective path are in contact or partially overlap.

Contrary to conventional reconstruction techniques in which one path of the X-ray imaging system is defined so as to acquire a set of 2D X-ray images having a sufficient number of images for complete reconstruction of the volume, the method according to the invention involves at least two paths of the X-ray imaging system, wherein each subset of 2D X-ray images acquired along the respective path is not sufficient alone to reconstruct the 3D image. In particular, an intersection between the cones of projection of each subset of images is smaller than the region of interest, whereas the intersection between the cones of projection of the images of all the subsets is generally greater than the region of interest.

In some embodiments, n is equal to 2.

In alternative embodiments, n is equal to 3.

The method may advantageously comprise a step of defining a virtual isocenter of the X-ray imaging system as being, for a same position of the X-ray source along the n paths, the center of a height of an enlarged cone formed by the union of the n cones of projection.

In some embodiments, the n trajectories are computed so as to place the virtual isocenter substantially at a center of the region of interest.

In other embodiments, the n trajectories are computed so as to minimize a distance between the virtual isocenter and a center of the region of interest.

At least two of the n paths may advantageously be implemented in opposite directions of orbital rotation of the X-ray imaging system.

Generally, an intersection between the cones of projection of each subset of images may be smaller than the region of interest.

In some embodiments, for a same position of the X-ray source along the n paths, the images of at least two of the n subsets partially overlap.

In some situations, a calibration phantom comprising radiopaque fiducials is detectable only in the image of one first subset, the method further comprising registering the image of at least one other subset to the image of the first subset based on the overlapping portion of said images.

In some embodiments, the method comprises selectively reducing the cone of projection of each 2D X-ray image using a dynamic collimator.

In preferred embodiments, the X-ray imaging system comprises a base, a C-shape gantry supporting the X-ray source and the image detector, and a motorized arm connecting the C-shaped-gantry to the base, the motorized arm presenting at least three rotation axes directed along a substantially common vertical direction.

Another object of the invention is a medical system for implementing the above-described method.

Said system comprises:
- an X-ray imaging system comprising an X-ray source and an image detector, and
- a control unit configured to:
   - compute n paths of the X-ray imaging system, n being an integer greater than or equal to 2, said paths being adapted to acquire n respective subsets of 2D X-ray images together forming the set of 2D X-ray images, each image of a subset defining a respective cone of projection of the region of interest onto the image detector, such that, for a same position of the X-ray source along the n paths, the cone of projection of an image of any subset is contiguous to the cone of projection of an image of at least one other subset;
   - control movement of the X-ray imaging system to execute the computed n paths to acquire the n subsets of images;
   - reconstruct the 3D medical image by intersecting the cones of projection defined by each image of the n subsets.

In preferred embodiments, the X-ray imaging system comprises a base, a C-shape gantry supporting the X-ray source and the image detector, and a motorized arm connecting the C-shaped-gantry to the base, the motorized arm presenting at least three rotation axes directed along a substantially common vertical direction.

### BRIEF DESCRIPTION OF THE FIGURES

Further features and advantages of the invention will be described in the following description, based on the appended drawings wherein:
- FIG. 1 is a schematic representation of an orbital path of a C-arm as commonly practiced in the prior art;
- FIG. 2 is a schematic representation of an X-ray imaging system that may be used in the present invention;
- FIG. 3 is a perspective view of an X-ray imaging system that may be used in the present invention;
- FIG. 4 schematically illustrates an enlarged cone based on the union of two adjacent cones of projection and the virtual isocenter of the X-ray imaging system for said enlarged cone;
- FIG. 5 schematically illustrates the first and second paths of the X-ray imaging system for a fixed position of the virtual isocenter of the X-ray imaging system, according to a first embodiment of the invention;
- FIG. 6A schematically illustrates the first path of the X-ray imaging system in the first embodiment;
- FIG. 6B schematically illustrates the second path of the X-ray imaging system in the first embodiment;
- FIG. 7 schematically illustrates the first and second paths of the X-ray imaging system for an elliptical trajectory of the virtual isocenter of the X-ray imaging system, according to a second embodiment of the invention;
- FIG. 8A schematically illustrates the first path of the X-ray imaging system in the second embodiment;
- FIG. 8B schematically illustrates the second path of the X-ray imaging system in the second embodiment;
- FIG. 9 schematically illustrates an enlarged cone based on the union of two partially overlapping cones of projection;
- FIG. 10 schematically illustrates an enlarged cone based on the union of two partially overlapping cones of projection, wherein a calibration phantom is visible in only of the corresponding 2D X-ray images;
- FIG. 11 schematically illustrates an enlarged cone based on the union of two adjacent cones of projection, wherein dynamic collimation has been used to reduce the irradiated area;
- FIG. 12 schematically illustrates an enlarged cone based on the union of three adjacent cones of projection;
- FIG. 13 illustrates an embodiment of the invention allowing increasing the size of the 3D image along the main axis of the patient.

For sake of legibility of the drawings, the figures are not necessarily drawn to scale.

The reference signs identical from one figure to another one designate the same elements or elements fulfilling the same function.

### DETAILED DESCRIPTION OF EMBODIMENTS

The invention may be implemented for reconstruction a 3D X-ray image in the context of a surgical intervention carried out onto one or several patient's bones, including but not limited to: implantation of orthopedic implants such as pedicular screws in the spine, implantation of various orthopedic implants in bones, reduction and fixation of fractures during traumatological procedures, positioning guides or canulae at a desired position with respect to a predefined target, or insertion of catheters or stents during cardio-vascular or urology procedures.

In this regard, the X-ray imaging system may be coupled to other surgical systems, such as a localization system and/or a surgical robotic system.

A region of interest corresponds to a volume of the patient's body to be acquired by an X-ray imaging technique. The region of interest may comprise a bone, a portion of a bone, or a plurality of adjacent bones.

### X-ray imaging system

As depicted in FIG. 2, the X-ray imaging system comprises at least one X-ray source S and at least one X-ray image detector D. The X-ray image detector may comprise a flat detector panel.

The X-ray source and X-ray image detector are carried by a C-shaped gantry G, the X-ray source and X-ray image detector being arranged on opposite ends of the gantry. Due to the shape of the gantry, such an imaging system is usually called a C-arm. As mentioned above, the center of the segment connecting the center of the X-ray source and the center of the detector is called the isocenter of the C-arm.

In the present invention, as in various conventional C-arms, the center of orbital motion of the C-arm is the isocenter. As a result, in the present text, the center of orbital motion and the isocenter designate the same point referred to as I.

In a manner known *per se,* the X-ray imaging system is configured to produce at least one 2D X-ray image that is the result of a conical projection of a region of interest onto the image detector, wherein the apex of the cone is approximately the central point of the X-ray source and the base of the cone is approximately the portion of the image detector that is reached by X-ray beams that have been collimated in a given shape and orientation.

A conventional C-arm is designed to allow the gantry to move relative to a base so as to move the X-ray source and detector about a patient while obtaining projection images of the patient placed between the X-ray source and the X-ray detector of the gantry.

For example, the X-ray imaging system may be a conventional C-arm, or any Cone-Beam Computed Tomography (CBCT) imaging system such as the SURGIVISIO device (ECENTIAL ROBOTICS, Gieres, France), or VISION FD VARIO 3D (ZIEHM), CIOS SPIN MOBILE 3D (SIEMENS), AIRO (STRYKER), LOOP-X (BRAINLAB), O-ARM (MEDTRONIC).

To reconstruct a 3D image, an imaging dataset comprising a plurality of 2D X-ray images of a region of interest is acquired by the X-ray imaging device. A 3D image of the region of interest can be reconstructed from the set of 2D X-ray images acquired by the X-ray imaging system using tomography techniques. The 3D image corresponds to the intersection of the cones of projection of the region of interest onto the detector corresponding to each 2D X-ray image of the imaging dataset.

In preferred embodiments, the X-ray imaging system may be motorized. In particular, the C-shaped gantry may comprise motors allowing movement horizontally (X and Y directions), vertically (Z direction) and around the X direction (defined by an angle a), so that 2D X-ray images of the patient may be produced from almost any angle. As shown on FIG. 2, the X axis is transversal to the operating table T on which the patient lies and the Y axis is parallel to the longitudinal axis of said operating table.

Each motor is associated to an encoder that provides at any time the relative position of the X-ray imaging system with respect to a reference position. When a 2D X-ray image is acquired, the corresponding position of the imaging system is recorded. Thus, each 2D image is recorded in the referential of the imaging system.

The path of the motorized C-arm is determined by each 2D X-ray image position of acquisition of said C-arm while performing a 3D image acquisition.

In some embodiments, the X-ray imaging system may comprise a mobile base allowing displacing the C-arm in the operating room. The C-shaped gantry may thus be slidably and/or pivotably mounted on said mobile base. The motors of the C-arm may be arranged in the mobile base and/or in the gantry.

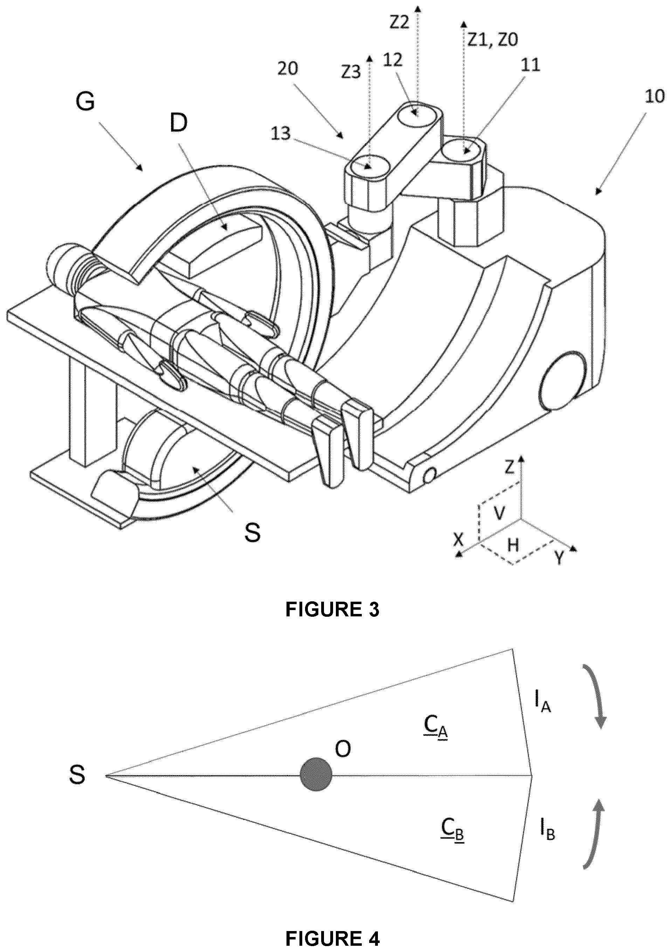

FIG. 3 illustrates such an embodiment of the X-ray imaging system, which comprises a mobile base 10, a motorized arm 20, and a C-shaped grantry G mounted on the mobile base 10 by means of the motorized arm 20.

The C-shaped gantry comprises an X-ray source S and an X-ray detector D.

The motorized arm 20 presents at least three rotation axes Z1, Z2, Z3 directed along a substantially common vertical direction Z. An example of such three rotation axes Z1, Z2, Z3 is illustrated in the example embodiment of figure 1.

A horizontal plane H is a plane normal to the vertical direction Z. The horizontal plane H is defined by a longitudinal direction Y and a transverse direction X orthogonal to the longitudinal direction Y.

A table on which a person to be imaged lies may extend in a substantially horizontal plane H. The longitudinal direction Y extends in a direction of a length of the table. The transverse direction X extends in a direction of a width of the table. A ground of the room in which the X-ray imaging is performed may extend in the horizontal plane H.

A vertical plane V corresponds to a plane defined by the vertical direction Z and the transverse direction X.

The movement of the C-arm 30 is managed by means of a single motorized arm 20, and is therefore simple.

The three rotation axes Z1, Z2, Z3 directed along a substantially common vertical direction Z are substantially parallel and articulate different segments of the motorized arm 20 relative to each other, so as to allow the motorized arm 20 to access quickly a wide range of positions in a wide variety of directions. The X-ray imaging system thus allows complex paths to be followed, which enables imaging of a large variety of regions of interests, under a large variety of constraints.

More particularly, the motorized arm 20 may move the C-shaped gantry G by a rotation or a combination of rotations of different segments of the motorized arm 20 around one or several of the three rotation axes Z1, Z2, Z3 of the motorized arm 20.

The motorized arm 20 may for example move the C-shaped gantry G in the horizontal plane H substantially normal to the vertical direction Z, for example in translation in the transverse direction X, the longitudinal direction Y, or any combination of directions comprised in the horizontal plane H.

A motorized arm 20 comprising three substantially parallel rotation axes Z1, Z2, Z3 also allows a high level of precision of the movement of the motorized arm 20, and therefore an accurate positioning and orienting of the C-arm 30.

The X-ray imaging system thus makes it possible to successively acquire distant imaging datasets of distant regions of interest, by moving the C-shaped gantry G between the successive acquisitions, while knowing that the movement command has been respected. The effective position of the C-shaped gantry G substantially corresponds to the commanded position of the C-shaped gantry G. Thus, the position of the X-ray source S and of the X-ray detector D are known for each successive acquisition of an imaging dataset.

Therefore, when several successive imaging datasets must be acquired, the relative position of the acquired images of a first dataset relative to the acquired images of a second dataset can be accurately be deduced from the commanded movement of the C-shaped gantry G. Therefore, the position of the C-shaped gantry G does not have to be recalibrated by specific recalibration means for the acquired images of different datasets.

The C-shaped gantry G may be positioned successively at different regions of interest, for example a foot, a knee, a hip, several vertebrae, etc. and/or acquire different datasets of images of a same region of interest, as will be described below. The position of the C-shaped gantry G may be deduced at any time from the commanded movement of the motorized arm 20, as the command is known to have been respected.

Finally, the base 10 is a mobile base. Therefore, the base 10 may be moved according to the region of interest to acquire, and may be moved in order to cooperate with a movement of the motorized arm 20. The mobile base 10 may be moved between successive acquisitions of different regions of interest. For example, the mobile base 10 may be positioned as close as possible to the region of interest to be acquired without hindering the doctor performing the X-ray imaging. This further reduces the complexity, weight and space needed by the X-ray imaging system.

This solution thus offers a greater flexibility in the acquisitions of imaging datasets with no loss of accuracy and with no significant impact on the weight and cost of the X-ray imaging system.

### Control unit

The X-ray imaging system is controlled by a control unit which typically comprises a processor, a data storage device and a communication device. In particular, said control unit controls the motors of the X-ray imaging system, i.e. the motors of the motorized C-arm and the motors of the motorized arm, when present.

The control unit may advantageously be embedded in the base of the X-ray imaging system. Said base may also comprise switches, such as a power switch, an emergency button and the like.

Alternatively, said control unit may be embedded in a separate cart with at least one interface with the C-arm, or may be remote, for example in a separate control room of the hospital or in a data center.

In preferred embodiments, the control unit may be able to control other surgical systems in the operating room.

The control unit is configured to implement suitable algorithms to carry out the workflow described below in order to reconstruct a 3D medical image.

### Workflow

The set of 2D X-ray images to be used for reconstruction of the 3D medical image is divided in two or more subsets of 2D X-ray images.

The acquisition of each subset of 2D X-ray images is carried out along a respective path of the X-ray imaging system. The determination of the path for each respective subset will be described below.

Each subset comprises a number of 2D X-ray images. This number may vary depending on the size and nature of the region of interest, but each subset is insufficient alone to reconstruct a 3D image of the region of interest. This means that, due to the specific design of the subsets, which will be described below, the intersection of the cones of projection of the images of each subset is not optimized and may be small or even void, and in any case is smaller than the region of interest.

By contrast, the combination of all the subsets comprises a sufficient number of 2D X-ray images with an optimized intersection - at the level of all the subsets - to allow reconstructing a 3D image of the region of interest. In particular, the intersection of the cones of projection of the images of all the subsets is computed to be greater than the region of interest.

Preferably, each subset comprises the same number of 2D X-ray images. In particular, the method forms a plurality of pairs, triplets or more generally groups of n 2D X-ray images, wherein n is an integer greater than or equal to 2, being the number of subsets, such that each 2D X-ray image of the group belongs to a respective distinct subset. Otherwise said, each image of a subset is associated with one image of each other subset to form such a group. It is to be that this association is only virtual, i.e. the n images of the group meet a requirement that will be described below, but the images are not physically assembled, stitched or otherwise linked to each other to reconstruct the 3D image.

More precisely, each group corresponds to a same position of the X-ray source along the n paths implemented to acquire the n subsets. The requirement to be met by the images of each group is that the cone of projection of an image of any subset is contiguous to the cone of projection of an image of at least one other subset. As mentioned above, "contiguous" means that two adjacent cones of projection of the group are in contact or partially overlap.

A first example of such a situation wherein n is equal to 2 is schematically illustrated in FIG. 4.

The X-ray source S has a common position between a pair of images I_{A} and I_{B}. Each image I_{A} and I_{B} belongs to a different subset.

The images I_{A} and I_{B} correspond to the projection of a respective cone C_{A}, C_{B} of X-rays generated by the source S onto the image detector during a respective path of the X-ray imaging system.

For example, as indicated by the arrows, the image I_{A} has been acquired along a first path of the X-ray imaging system according to a clockwise orbital rotation about its isocenter, and the image I_{B} has been acquired along a second path of the X-ray imaging system according to a counter-clockwise orbital rotation of the X-ray imaging system about its isocenter. Implementing the first and second paths according to opposite directions of rotation is advantageous to optimize the time and whole path of acquisition of the set of 2D X-ray images. In particular, implementing the first and second paths in opposite directions allows avoiding returning to a starting position of the C-arm between the two paths, which saves time; in addition, the movements of the C-arm are minimized, which may be beneficial to reduce errors in the positioning of the cones of projection due to lack of accuracy of the mechanical components of the X-ray imaging system.

In the illustrated embodiment, the cones of projection C_{A}, C_{B} share a common boundary and are thus contiguous.

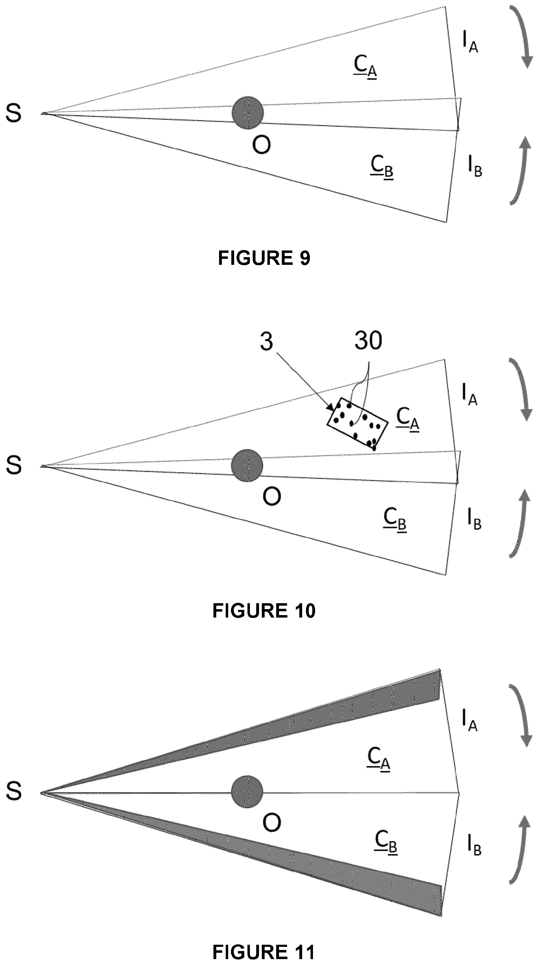

Alternatively, the cones of projection may partially overlap and are thus also considered to be contiguous within the meaning of the present disclosure. This situation is schematically illustrated in FIG. 9.

The X-ray source S has a common position between a pair of images I_{A} and I_{B}. Each image I_{A} and I_{B} belongs to a different subset.

The images I_{A} and I_{B} correspond to the projection of a respective cone C_{A}, C_{B} of X-rays generated by the source S onto the image detector during a respective path of the X-ray imaging system.

For example, as indicated by the arrows, the image I_{A} has been acquired along a first path of the C-arm according to a clockwise orbital rotation about its isocenter, and the image I_{B} has been acquired along a second path of the C-arm according to a counter-clockwise orbital rotation of the C-arm about its isocenter.

The cones of projection C_{A}, C_{B} partially overlap and share a common area corresponding to their intersection.

An advantage of such an overlapping is to avoid having gaps between the cones of projection, for example due to lack of accuracy of the mechanical components of the X-ray imaging system.

In addition, a greater quantity of X-rays passes through the region of interest in the overlapping portion, thereby providing a greater accuracy of the reconstructed 3D image in this portion.

Another advantage of such an overlapping is that registration of the images I_{A} and I_{B} can be carried out based on the elements of the region of interest present in the intersecting portion of said images. Such a registration is all the more advantageous that the images I_{A} and I_{B} may be acquired at different times, such that the region of interest may have moved relative to the X-ray source. For example, the patient's breathing may generate a cyclic movement of the region of interest, such that, if the images I_{A} and I_{B} are not acquired at a same stage of the breathing cycle, the position of the region of interest is not the same in both images. The above-mentioned registration based on the overlapping portion of the images allows correcting this offset.

In preferred embodiments, a phantom is used to calibrate the 3D geometry of the X-ray imaging system at the same time as the image acquisition. As shown in FIG. 10, said phantom 3 comprises a plurality of radiopaque fiducials 30 arranged according to a known geometry. The radiopaque fiducials can thus be detected in a 2D X-ray image of a portion of the patient's body comprising the phantom. However, said phantom is usually rather small and compact, and may thus be visible in only some of the 2D X-ray images. In each group, the image(s) wherein the phantom is visible (e.g. image I_{A} in the figure) can then be registered with the image(s) wherein the phantom is not visible (e.g. image I_{B} in the figure) using the overlapping portion of the images.

In some embodiments, the X-ray imaging system may comprise dynamic collimators adapted to reduce the areas of the X-ray beam generated by the X-ray source. In the present method, said dynamic collimators can be adjusted for each cone of projection to protect some regions of the patient's body that must not or need not be irradiated. Such collimation is schematically illustrated in FIG. 11, in which the grey areas in cones C_{A} and C_{B} represent regions that are not irradiated by the X-rays. By selecting dynamic collimation on each position of acquisition of the X-ray imaging system (which may be different for each position), and two or more paths of image acquisition, it is possible to reconstruct a 3D image conforming to many desired geometries avoiding irradiating selected regions of the patient's body. In addition, alternating clockwise and anticlockwise paths makes it possible to generate such geometry in a reasonable time frame since the X-ray imaging system is always moving at a desired speed to collect the necessary images.

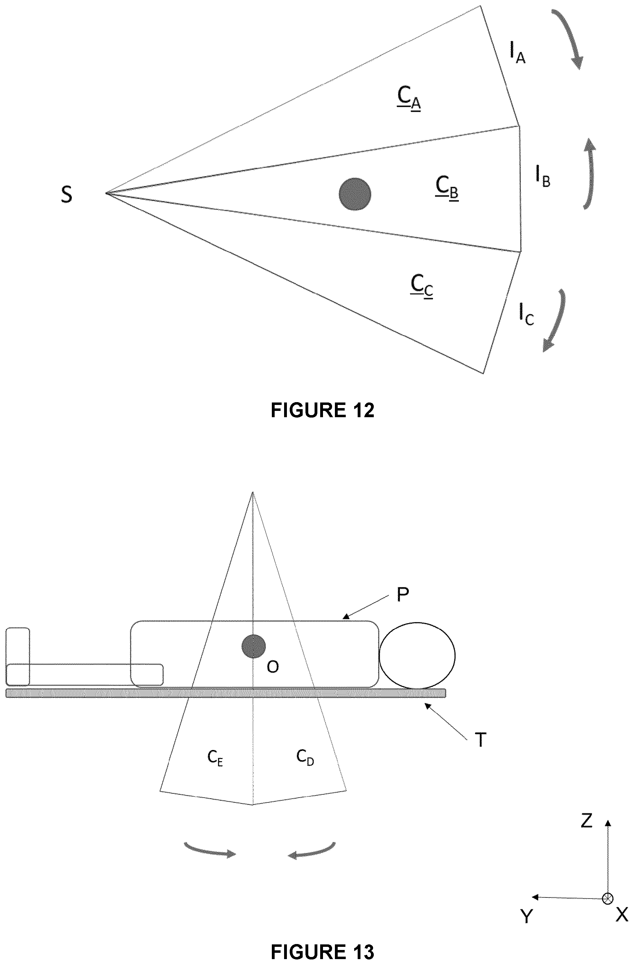

Another example, for a situation wherein n is equal to 3, is schematically illustrated in FIG. 12.

The X-ray source S has a common position between a triplet of images I_{A}, I_{B} and I_{C}. Each image I_{A}, I_{B} and I_{C} belongs to a different subset. The images I_{A}, I_{B} and I_{C} correspond to the projection of a respective cone C_{A}, C_{B} and Cc of X-rays generated by the source S onto the image detector during a respective path of the X-ray imaging system. For example, as indicated by the arrows, the image I_{A} has been acquired along a first path of the C-arm according to a clockwise orbital rotation about its isocenter, the image I_{B} has been acquired along a second path of the C-arm according to a counter-clockwise orbital rotation of the C-arm about its isocenter, and the image I_{C} has been acquired along a third path of the C-arm according to a clockwise orbital rotation about its isocenter.

Although the images I_{A}, I_{B} and I_{C} are represented with a common boundary two by two, there may be an overlap between images I_{A} and I_{B} and between images I_{B} and I_{C}, as described above. In this way, it is possible to register the three images together based on their overlapping portions.

Of course, the number n of subsets may be greater than 3, although it requires a greater number of paths of the X-ray imaging system.

The union of the n images of each group can be seen as an enlarged cone of projection of X-rays generated by the source passing through the region of interest onto a virtual image detector.

The intersection of all the enlarged cones obtained with the plurality of paths of the X-ray imaging system is greater than the intersection of cones of projection of 2D X-ray images acquired in a path of the X-ray imaging system not allowing forming groups of contiguous images. As a result, the size of the reconstructed 3D image is increased. As mentioned previously, the reconstruction of the 3D image does not require assembling, stitching or otherwise physically linking the 2D X-ray images of each group. The reconstruction of the 3D image conventionally requires intersecting the cones of projection corresponding to each 2D X-ray image of the whole set of images, but, thanks to the requirement on the contiguity of the images of each group, the volume resulting from said intersection is greater than with conventional paths of the X-ray imaging system.

As shown in FIGS. 4, 9 and 12 described above, the apex of the enlarged cone resulting from the union of the cones of projection of each group of 2D X-ray images is the central point of the X-ray source S and the base of the enlarged cone defines the virtual image detector. It is thus possible to define a virtual isocenter O of the X-ray imaging system as being the center of the height of said enlarged cone, i.e. the distance between the X-ray source and the virtual image detector.

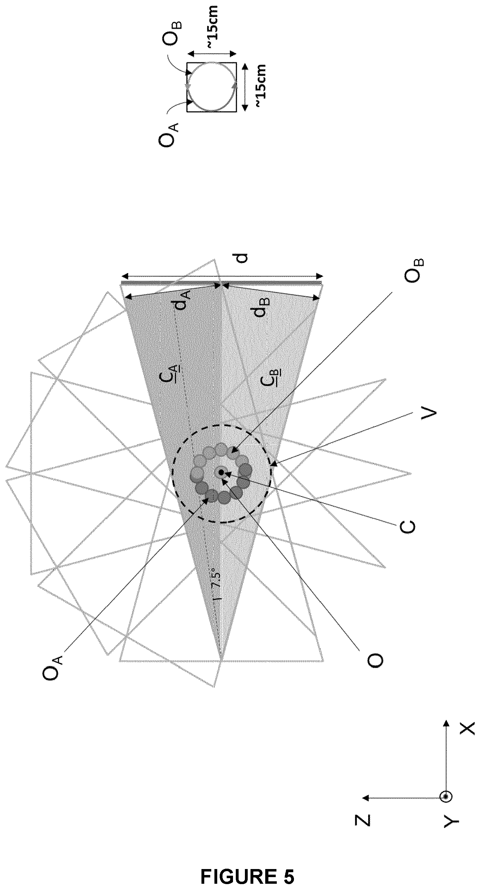

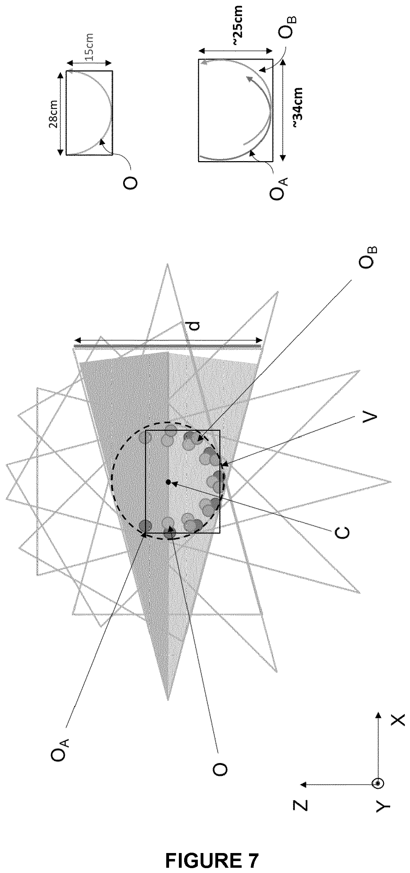

Advantageously, to optimize the size of the 3D image, the virtual isocenter has to be placed substantially at the center C of the region of interest (see FIG. 5), or at a minimal distance from the center C of the region of interest (see FIG. 7).

The position of the center of the region of interest relative to the X-ray imaging system can be determined or at least estimated in various ways. In some embodiments, a phantom comprising a set of radiopaque fiducials in a known spatial configuration may be placed within the volume to be imaged and in such a way that the spatial relationship between said radiopaque fiducials (or any other fixed reference) and the center of the region of interest is known and fixed. Generally, the center of the region of interest relative to the patient's body is at least approximately known based on anatomical knowledge of the patient's body. Alternatively, the center of the region of interest may be defined by an input of a physician on preoperative images or on intraoperative images acquired prior to the acquisition paths for reconstructing the 3D image. For example, two 2D X-ray images may be acquired with the X-ray imaging system, allowing for a 3D positioning of the center of the region of interest. For example, said two images may be a frontal and a lateral view of the region of interest to be imaged. In other embodiments, the center of the region of interest may be determined automatically by image analysis. In any case, the position of the center of the region of interest is thus determined in a coordinate system of the X-ray imaging system. In such a way, the control unit is able to move the C-arm with respect to the center of the region of interest.

The path corresponding to each subset of 2D X-ray images may thus be computed so that, for each position of the X-ray source, the virtual isocenter is located at or near the center of the region of interest.

FIG. 5 schematically illustrates the first and second paths of the X-ray imaging system for a fixed position of the virtual isocenter of the X-ray imaging system, for example corresponding to the position of the center C of the region of interest, in a case where n is equal to 2 (see FIG. 4). Otherwise said, the virtual image detector has an orbital motion about the virtual isocenter. Each path is designed to acquire a respective subset of 2D X-ray images, such that each image of the subset corresponds to a cone of projection contiguous to the cone of projection of an image of the other subset. In each path, the real isocenter of the C-arm (i.e. the center of the segment which links the X-ray source S to the center of the detector panel) moves to meet the above requirement on the position of the virtual isocenter.

The points designated by O_{A} represent the position of the real isocenter of the C-arm during the first path and the points designated by O_{B} represent the position of the real isocenter of the C-arm during the second path.

One group of contiguous cones of projection C_{A}, C_{B} is represented. The cones of projection for other positions of the C-arm along the first and second paths are represented with grey boundaries. The dotted contour surrounding the virtual isocenter O represents the volume V of the reconstructed 3D image, defined by the intersection of the plurality of cones.

d_{A} and d_{B} represent the width of the base of the respective cone C_{A}, C_{B}, which is also the size of the respective 2D X-ray image. d represents the width of the base of the enlarged cone formed by the union of cones C_{A}, and C_{B}.

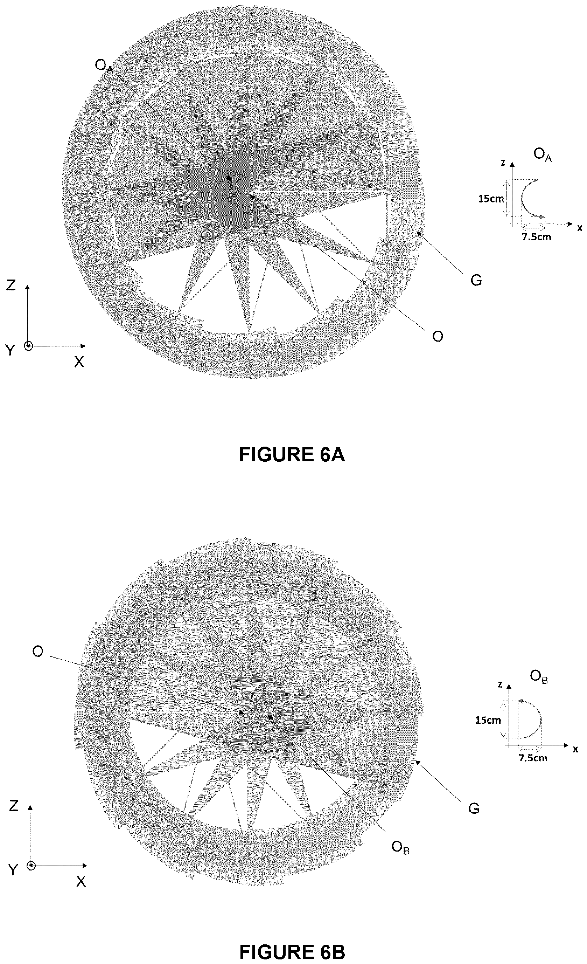

FIG. 6A and 6B schematically illustrate the first and second trajectories of the real isocenter of the C-arm to obtain the volume V represented in FIG. 5.

To reconstruct a volume V having a diameter d of about 30 mm, the real isocenter of the C-arm has to move in translation by 7.5 cm in the x direction and by 15 cm in the z direction in both paths. The angular displacement of the virtual image detector is from 172.5° to -7.5° for the first path, and from 187.5° to 7.5° for the second path.

The trajectory of the virtual image detector may also be more complex than a pure orbital motion.

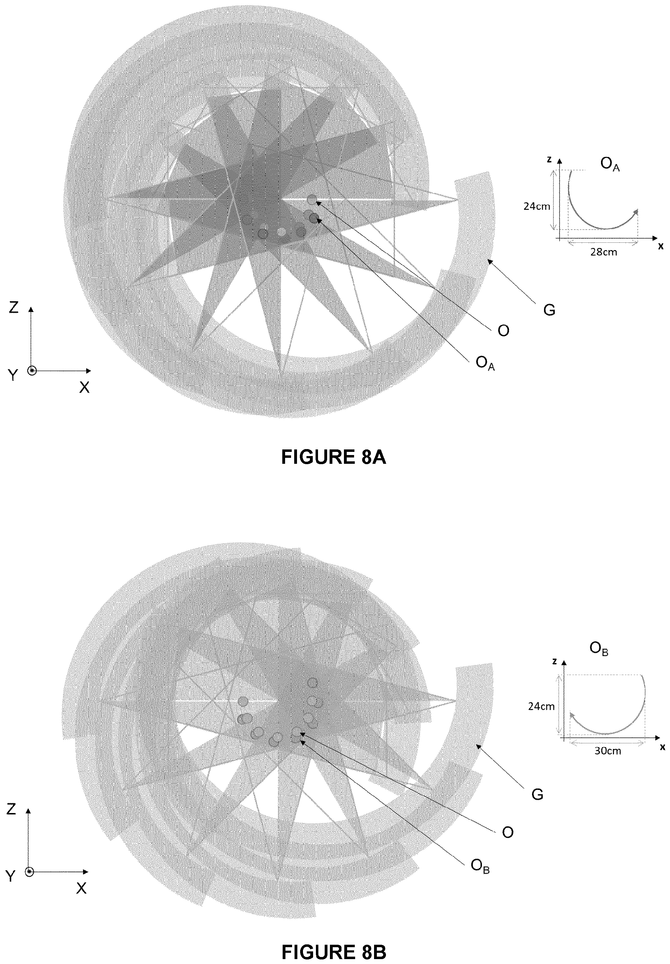

In some embodiments, as illustrated in FIGS. 7 and 8A-8B, the virtual isocenter O may have an elliptical trajectory adapted to minimize the distance between the virtual image detector and the center C of the region of interest for each position of the X-ray source. Such a trajectory allows increasing the size of the reconstructed 3D image.

For example, to reconstruct a volume V having a diameter d of about 38 mm, the virtual isocenter has to move in translation by 28 cm in the x direction and by 15 cm in the z direction. The real isocenter of the C-arm has to move in translation by 28 cm in the x direction in the first path (30 cm in the second path) and by 24 cm in the z direction in both paths. The angular displacement of the virtual image detector is from 172.5° to -7.5° for the first path, and from 187.5° to 7.5° for the second path.

If appropriate, other types of paths can be defined.

In particular, the method described above can also be used to increase the size of the reconstructed image in the Y direction, and not only in the X, Z directions as described in the previous embodiments.

To that end, as shown in FIG. 13, two paths of the X-ray imaging system can be computed to acquire two subsets of 2D X-ray images using rotations about the α angle of the C-arm and translation along the Y axis. For a same position of the X-ray source along both paths, the cone of projection C_{D} of an image of the first subset is contiguous to the cone of projection C_{E} of an image of the second subset. As described previously, the first path can be implemented clockwise and the second path can be implemented counter-clockwise.

Preferably, said paths are combined to the paths described above, so as to expand the size of the reconstructed 3D image in X, Y and Z directions. This allows, in particular, imaging an elongated anatomical structure, such as the patient's spine.

The X-ray imaging system described with reference to FIG. 3 is particularly advantageous to implement the method of the present disclosure, due to (i) its great mechanical accuracy and (ii) the large amplitude of displacement provided by the motorized arm 20.

Of course, as described previously, more than two paths may be used; in addition, the cones of projection may not simply share a boundary but partially overlap.

After the n paths have been computed, the control unit controls the C-arm to implement each path and acquire a subset of 2D X-ray images during each path.

Then, the control unit computes the reconstruction of the 3D image based on the 2D X-ray images of the n subsets. To that end, the control unit implements algorithms that are known by the skilled person.

The resulting 3D image has thus a greater size than a 3D image reconstructed from any conventional path of acquisition of the X-ray imaging system.

It is to be noted that the present disclosure is not limited to the illustrated and above-described embodiments. In particular, the individual embodiments can be combined whenever such a combination is technically feasible.

## Claims

1. Method for reconstructing a 3D medical image of a region of interest from a set of 2D X-ray images acquired by an X-ray imaging system comprising an X-ray source (S) and an image detector (D), comprising the steps of:
- computing n paths of the X-ray imaging system, n being an integer greater than or equal to 2, said paths being adapted to acquire n respective subsets of 2D X-ray images together forming the set of 2D X-ray images, each image (I_{A}, I_{B}, Ic) of a subset defining a respective cone of projection (C_{A}, C_{B}, Cc) of the region of interest onto the image detector, such that, for a same position of the X-ray source (S) along the n paths, the cone of projection of an image of any subset is contiguous to the cone of projection of an image of at least one other subset;
- implementing the computed n paths to acquire the n subsets of images;
- reconstructing the 3D medical image by intersecting the cones of projection defined by each image of the n subsets.

2. Method according to claim 1, wherein n is equal to 2.

3. Method according to claim 1, wherein n is equal to 3.

4. Method according to any one of claims 1 to 3, further comprising defining a virtual isocenter (O) of the X-ray imaging system as being, for a same position of the X-ray source along the n paths, the center of a height of an enlarged cone formed by the union of the n cones of projection.

5. Method according to claim 4, wherein the n trajectories are computed so as to place the virtual isocenter (O) substantially at a center (C) of the region of interest.

6. Method according to claim 4, wherein the n trajectories are computed so as to minimize a distance between the virtual isocenter (O) and a center (C) of the region of interest.

7. Method according to any one of claims 1 to 6, wherein at least two of the n paths are implemented in opposite directions of orbital rotation of the X-ray imaging system.

8. Method according to any one of claims 1 to 7 wherein an intersection between the cones of projection of each subset of images is smaller than the region of interest.

9. Method according to any one of claims 1 to 8, wherein, for a same position of the X-ray source along the n paths, the images of at least two of the n subsets partially overlap.

10. Method according to claim 9, wherein a calibration phantom (3) comprising radiopaque fiducials (3) is detectable only in the image of one first subset, the method further comprising registering the image of at least one other subset to the image of the first subset based on the overlapping portion of said images.

11. Method according to any one of claims 1 to 10, further comprising selectively reducing the cone of projection of each 2D X-ray image using a dynamic collimator.

12. Method according to any one of claims 1 to 11, wherein the X-ray imaging system comprises a base (10), a C-shape gantry (G) supporting the X-ray source (S) and the image detector (D), and a motorized arm (20) connecting the C-shaped-gantry (G) to the base (10), the motorized arm (20) presenting at least three rotation axes (Z1, Z2, Z3) directed along a substantially common vertical direction (Z).

13. Medical system for reconstructing a 3D medical image of a region of interest, comprising:
- an X-ray imaging system comprising an X-ray source (S) and an image detector (D), and
- a control unit configured to:
- compute n paths of the X-ray imaging system, n being an integer greater than or equal to 2, said paths being adapted to acquire n respective subsets of 2D X-ray images together forming the set of 2D X-ray images, each image (I_{A}, I_{B}, Ic) of a subset defining a respective cone of projection (C_{A}, C_{B}, Cc) of the region of interest onto the image detector, such that, for a same position of the X-ray source (S) along the n paths, the cone of projection of an image of any subset is contiguous to the cone of projection of an image of at least one other subset;
- control movement of the X-ray imaging system to execute the computed n paths to acquire the n subsets of images;
- reconstruct the 3D medical image by intersecting the cones of projection defined by each image of the n subsets.

14. Medical system according to claim 13, wherein the X-ray imaging system comprises a base (10), a C-shape gantry (G) supporting the X-ray source (S) and the image detector (D), and a motorized arm (20) connecting the C-shaped-gantry (G) to the base (10), the motorized arm (20) presenting at least three rotation axes (Z1, Z2, Z3) directed along a substantially common vertical direction (Z).
